# EUROPEAN PATENT SPECIFICATION

(11) **EP 2 776 821 B1**
(45) Date of publication and mention of the grant of the patent: **08.01.2020**
(21) Application number: 12791360.6
(22) Date of filing: 06.11.2012
(51) Int. Cl.: G01N 27/447, G01N 33/543

(54) **FLUIDIC DEVICES HAVING INCORPORATED ELECTRODES**
FLUIDISCHE VORRICHTUNGEN MIT DARIN EINGESETZTEN ELEKTRODEN
DISPOSITIFS FLUIDIQUES COMPORTANT DES ÉLECTRODES INCORPORÉES

(30) Priority: 07.11.2011 US 201113290843
(43) Date of publication of application: 17.09.2014
(73) Proprietor: Caliper Life Sciences, Inc., Alameda, CA 94501 (US)
(72) Inventor: KENNEDY, Colin B., Greenbrae, California 94904 (US); MOLHO, Josh, Oakland, California 94619 (US); DUKHOVNY, Alexander V., San Francisco, California 94127 (US)
(74) Representative: Mewburn Ellis LLP
(86) International application number: PCT/US2012/063755
(87) International publication number: WO 2013/109334

(56) References cited:
- FR-A1- 2 897 858
- US-A1- 2004 203 055
- US-A1- 2011 139 274
- US-A1- 2012 080 315

## Description

### TECHNICAL FIELD

The present disclosure is in the field of devices, systems, and methods for processing, separating, isolating, and/or analyzing sample components. In particular, described herein are fluidic devices having incorporated electrodes, systems for using such devices, and methods for manufacturing such devices. The invention also provides fluidic devices configured to absorb joule heating within the device.

### BACKGROUND OF THE INVENTION

Separations-based analyses are a prominent part of biological research, allowing one to characterize different biological samples, reaction products and the like. Examples of some of the more prevalent separations-based analyses include electrophoretic separations of macromolecular species, e.g., proteins and nucleic acids. Electrophoresis, e.g., capillary electrophoresis, has been established as a highly effective method for separating macromolecular species in order that they might be further characterized. Protein and nucleic acid molecules are two major examples of molecular species that are routinely fractionated and characterized using electrophoretic systems.

Both microfluidic and macrofluidic devices have been applied in separations-based analyses. Examples of novel microfluidic devices and methods for use in the separation of molecular and macromolecular species by electrophoretic means are described in United States Patent Nos. 5,958,694, 6,032,710, 2011/139274, 2012/080315 and 7,419,784, for example. In such devices, the sample containing the molecular or macromolecular species for which separation is desired is placed in one end of a separation channel located in a fluidic substrate, and a voltage gradient is applied along the length of the channel. As the sample components are electrophoretically transported along the length of the channel and, optionally, through a separation (sieving) matrix disposed therein, those components are resolved. The separated components are then detected at a detection point along the length of the channel, typically near the terminus of the separation channel downstream from the point at which the sample was introduced. Following detection, the separated components may be directed to a collection reservoir/well in the device (or to an external device such as a multiwell plate using a capillary pipettor, for example) for subsequent extraction or disposal.

In many situations, it is desirable to extract selected fragments of interest, such as DNA (deoxyribonucleic acid) fragments, following the separation of the fragments into bands in the separation matrix for further processing or analysis, e.g., restriction enzyme modification, T4 ligation, PCR (polymerase chain reaction) amplification, mass spectroscopy, or polynucleotide kinase reactions. The typical process used by laboratory researchers for extracting and isolating selected DNA fragments of interest (and other desired nucleic acid and protein fragments) from a separation matrix (such as an agarose gel) involves staining the separated fragments and then shining UV (ultraviolet) light on the fragments to visualize the separated bands. A razor blade is then used to manually cut the gel above and below each fragment of interest. The DNA must then be extracted and purified from the gel slice. The recovered DNA can then be used for further processing or analysis. This extraction process, however, is time consuming, laborious, and potentially damaging to the DNA (e.g., nicking of the DNA can occur if the DNA is exposed to UV light too long while the fragments of interest are being illuminated for excision).

Thus, in performing separations-based analyses, it would be desirable to be able to also isolate or extract one or more of the separated components in the device itself for further analysis or processing. The recovered or isolated fragments could then be used for a variety of different processes including, for example, the following: amplification using polymerase chain reaction (PCR); ligation reactions for cloning small to medium-sized strands of DNA into bacterial plasmids, bacteriophages, and small animal viruses to allow the production of pure DNA in sufficient quantities to allow its chemical analysis; reactions to dissolve a separated protein or nucleic acid component in a suitable matrix for further analysis by a mass spectrometer using, for example, Matrix-Assisted Laser Desorption Ionization (MALDI); binding reactions to bind a labeling agent to one or more separated protein or nucleic acid components for further analysis; or other similar post-detection processes. In addition, in the case of PCR samples, it is important to be able to separate smaller dimer and primer molecules from the main nucleic acid fragments in the sample and then isolate and collect the main nucleic acid fragments for further analysis or processing, while directing the smaller primer and dimer components to a waste reservoir/well for removal and subsequent disposal.

Typically, the electrodes used in performing electrophoretic separations in a fluidic device are included in an instrument that receives the fluidic device, rather than in the fluidic device itself. Because the instrument electrodes are not disposable and are used serially with multiple devices, contamination of the samples, reaction mixtures, and reaction products may result. In some separations-based applications, limited contamination is not a problem; however, when a component of interest is to be isolated or extracted from a sample, avoiding contamination of a subsequent sample with components of an earlier sample can be crucial. Therefore, it would be advantageous to provide improved fluidic devices that incorporate electrodes into the devices, eliminating the need for multi-use electrodes being included in the instrument that receives the devices. US 8,367,210 B2 discloses fluidic devices having incorporated electrodes.

### SUMMARY OF THE INVENTION

Disclosed herein is a fluidic device having incorporated electrodes. A first device comprises a card and a caddy. The card comprises a channel having first and second ends, the channel disposed within the card. The card further comprises first and second vias in fluid communication with the channel through an upper surface of the card and first and second electrodes disposed on the upper surface of the card. The vias in the top portion of the card are oriented such that they are in communication with at least one of the channels and/or chambers formed in the interior portion of the device. The first via and first electrode are positioned adjacent to the first end of the channel, and the second via and second electrode are positioned adjacent to the second end of the channel. The caddy comprises first and second reservoirs. The caddy is attached to the card such that the first reservoir is positioned over the first via and a portion of the first electrode, the second reservoir is positioned over the second via and a portion of the second electrode, and a portion of each of the first electrode and the second electrode is accessible for dry electrical contact.

Another fluidic device having incorporated electrodes comprises a card and a caddy. The card comprises a channel having first and second ends, the channel disposed within the card. The card further comprises a first via in fluid communication with the channel through an upper surface of the card, the first via positioned adjacent to the first end of the channel, and a second via in fluid communication with the channel through the upper surface of the card, the second via positioned adjacent to the second end of the channel. The caddy comprises first and second reservoirs and first and second electrodes. The first electrode is positioned such that a first portion of the first electrode extends into the first reservoir and a second portion of the first electrode is accessible for dry electrical contact, and the second electrode is positioned such that a first portion of the second electrode extends into the second reservoir and a second portion of the second electrode is accessible for dry electrical contact. The caddy is attached to the card such that the first reservoir is positioned over the first via and the second reservoir is positioned over the second via. The electrodes may be disposed on deformable tabs or they may be fabricated as structures independent of the caddy and inserted into openings in the caddy.

Also disclosed herein is a fluidic device configured to absorb joule heating within the device, the device comprising a card and a caddy. The card comprises a channel having first and second ends, the channel disposed within the card. The card further comprises first and second vias in fluid communication with the channel through an upper surface of the card. The first via is positioned adjacent to the first end of the channel, and the second via is positioned adjacent to the second end of the channel. The caddy comprises first and second reservoirs. The caddy is attached to the card such that the first reservoir is positioned over the first via and the second reservoir is positioned over the second via. The first reservoir extends over an area covering substantially a first longitudinal half of the channel, and the second reservoir extends over an area covering substantially a second longitudinal half of the channel.

Also disclosed herein is a system for isolating one or more sample components of a sample material following separation of the sample material into a plurality of sample components. The system comprises a device having incorporated electrodes such as has been described herein, a detector in sensory communication with the device, a fluid direction system, and a processor operably coupled to the detector and the fluid direction system.

Also disclosed herein is a method of manufacturing a fluidic device having incorporated electrodes. A first method comprises providing a card, providing a caddy, and attaching the caddy to the card. The card comprises a channel, first and second vias, and first and second electrodes. The channel is disposed within the card and has first and second ends. The first and second vias are in fluid communication with the channel through an upper surface of the card. The first and second electrodes are disposed on the upper surface of the card. The first via and first electrode are positioned adjacent to the first end of the channel, and the second via and second electrode are positioned adjacent to the second end of the channel. The caddy comprise first and second reservoirs. The caddy is attached to the card such that the first reservoir is positioned over the first via and a portion of the first electrode, the second reservoir is positioned over the second via and a portion of the second electrode, and a portion of each of the first electrode and the second electrode is accessible for dry electrical contact.

A second method comprises providing a card, providing a caddy, and attaching the caddy to the card. The card comprises a channel and first and second vias. The channel is disposed within the card and has first and second ends. The first via is in fluid communication with the channel through an upper surface of the card and is positioned adjacent to the first end of the channel. The second via is in fluid communication with the channel through the upper surface of the card and is positioned adjacent to the second end of the channel. The caddy comprises first and second reservoirs and first and second electrodes. The first electrode is positioned such that a first portion of the first electrode extends into the first reservoir and a second portion of the first electrode is accessible for dry electrical contact, and the second electrode is positioned such that a first portion of the second electrode extends into the second reservoir and a second portion of the second electrode is accessible for dry electrical contact. The caddy is attached to the card such that the first reservoir is positioned over the first via and the second reservoir is positioned over the second via.

The aforementioned and other features and advantages of the invention will become further apparent from the following detailed description of the presently preferred embodiments, read in conjunction with the accompanying drawings, which are not to scale. In the drawings, like reference numbers indicate identical or functionally similar elements. The detailed description and drawings are merely illustrative of the invention, rather than limiting, the scope of the invention being defined by the appended claims.
The invention is defined in claims 1 and 17. Preferred features are set out in the dependent claims.

### BRIEF DESCRIPTIONS OF THE DRAWINGS

**FIGS. 1A** and **1B** are an exploded view and an assembled view, respectively, of a multilayer fluidic card with incorporated electrodes, in accordance with the present disclosure;
**FIGS. 2A** and **2B** are schematic illustrations of the multilayer fluidic card of **FIG. 1** positioned adjacent to a caddy containing reservoirs, in accordance with the present disclosure; **FIG. 2A** showing the card uppermost and **FIG. 2B** showing the caddy uppermost;
**FIG. 3A** is a top view of the multilayer fluidic card and caddy illustrated in **FIGS. 2A** and **2B** assembled into a fluidic device; **FIGS. 3B** and **3C** show cross-sectional views of the fluidic device of **FIG. 3A,** and **FIG. 3D** shows an enlarged view of a portion of **FIG. 3B****;**
**FIGS. 4A** and **4B** are an exploded view and an assembled view, respectively, of another fluidic device having incorporated electrodes, in accordance with the present disclosure;
**FIGS. 5A, 5B,** and **5C** are a top view, a bottom view, and a cross-sectional view, respectively, of the fluidic device of **FIGS. 4A** and **4B****,** in accordance with the present disclosure;
**FIGS. 6A** and **6B** are a bottom view and an angled top view, respectively, of a channel sheet, in accordance with the present disclosure;
**FIGS. 7A, 7B, 7C,** and **7D** are a top view, a bottom view, an angled bottom view, and an angled top view, respectively, of the caddy of **FIGS. 4A, 4B****,** and **5A****-5C,** in accordance with the present disclosure, the caddy having incorporated electrodes;
**FIGS. 8A** and **8B** are an exploded view and a top view, respectively, of the patterned/punched label also seen in **FIG. 4A****,** in accordance with the present disclosure;
**FIGS. 9A** and **9B** are an exploded view and an assembled view, respectively, of yet another fluidic device having incorporated electrodes, in accordance with the present disclosure;
**FIGS. 10A** and **10B** are a top view and a bottom view, respectively, of the fluidic device of **FIGS. 9A** and **9B****,** while **FIGS. 10C** and **10D** are cross-sectional views of the same fluidic device; and
**FIGS. 11A** and **11B** are exploded views shown from the top and bottom, respectively, of a fluidic device in accordance with the present invention.

### DETAILED DESCRIPTION OF THE

### PRESENTLY PREFERRED EMBODIMENTS

The disclosure provides a fluidic device having incorporated electrodes. The fluidic device comprises a multilayer fluidic card and a caddy. The electrodes of the fluidic device may be incorporated into either the card or the caddy.

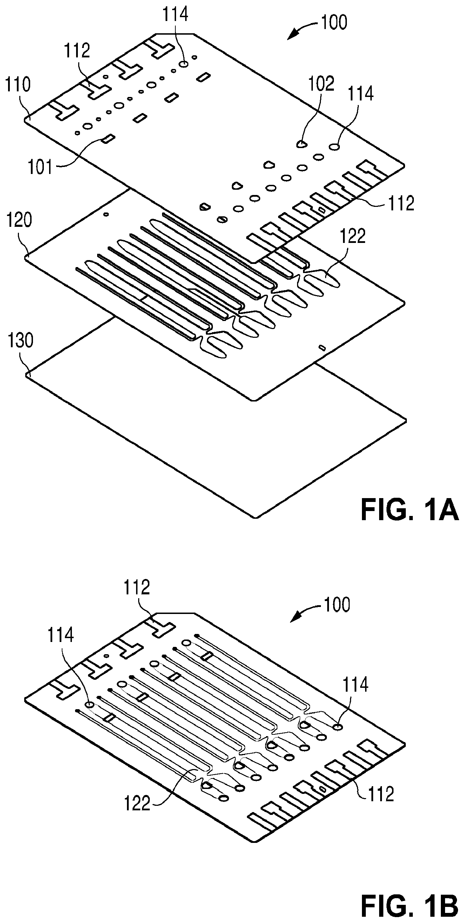

One embodiment of a fluidic device having incorporated electrodes, in accordance with the present disclosure, is illustrated in **FIGS. 1A-3D****.** The device comprises a multilayer fluidic card **100** and a caddy **200.** In the present embodiment, the electrodes are incorporated into the multilayer card. Card **100** comprises a top sheet **110,** a channel sheet **120,** and a bottom sheet **130.** Top sheet **110** includes wells **101** and **102,** electrodes **112,** and vias **114.** Channel sheet **120** includes channels **122.** Caddy **200** includes wells **201** and **202** and reservoirs **205.**

**FIG. 1A** shows an exploded view of multilayer card **100,** while **FIG. 1B** shows the card assembled. As seen in **FIG. 1A****,** top sheet **110** includes a plurality of electrically isolated electrodes **112** disposed on a top surface of the top sheet and, therefore, disposed on a top surface of the multilayer card. While eight electrodes are shown in **FIGS. 1A** and **1B** positioned on two edges of a substantially rectangular sheet, the number and positioning of the electrodes may vary as may the shape of the card. In the present embodiment, electrodes **112** are fabricated using a carbon conductive paste. In alternative embodiments, the electrodes may comprise different or additional materials, including, for example, gold, copper, platinum, silver/silver chloride paste, and other conductive materials. The electrodes may be patterned onto top sheet **110** by screen printing, pad printing, ink-jet printing, stenciling, or other similar methods. Alternatively, the electrodes may be formed separately and attached to the sheet using methods known in the art.

The electrodes may be fabricated using a single material or multiple materials. For example, multiple materials may be used where one of the electrode materials has high conductivity but is incompatible with liquids to be used in and with the device. In this case, electrodes may be fabricated by applying a chemically compatible material (e.g., a carbon conductive paste) to regions of the device that will come into contact with a liquid, applying a second material (e.g., a high-conductivity material such as silver) outside of the regions of the device that contact a liquid, and connecting the second material to the chemically compatible material. Alternatively, a chemically incompatible conductive material may be applied first to top sheet **110** and then covered completely with a chemically compatible conductive material. In this way, low electrical resistance is achieved, but a liquid within the device is exposed only to a chemically compatible conductive material.

Each via **114** extends through top sheet **110** to provide fluid communication with at least one channel **122** formed in channel sheet **120.** Thus, the configuration of vias **114** in top sheet **110** is based on the configuration of the channel or channels **122** in channel sheet **120.** While one configuration of vias and channels is illustrated in the figures, other configurations are possible. Vias **114** and channels **122** may be formed using techniques known in the art such as molding, etching, drilling, laser cutting, and punching.

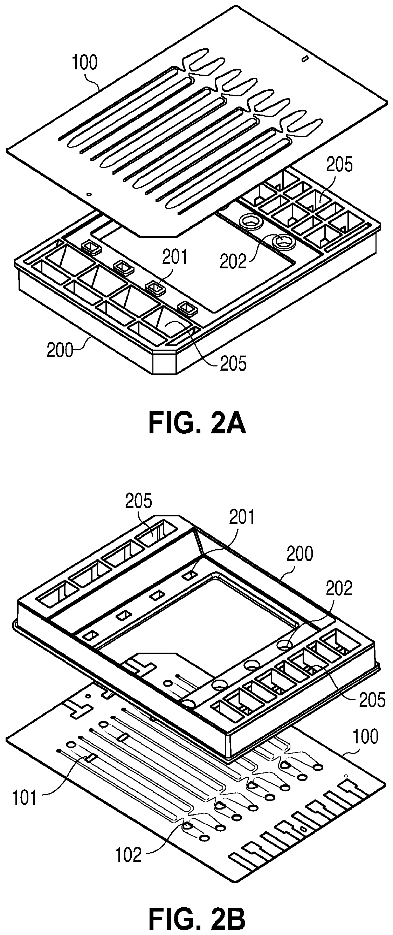

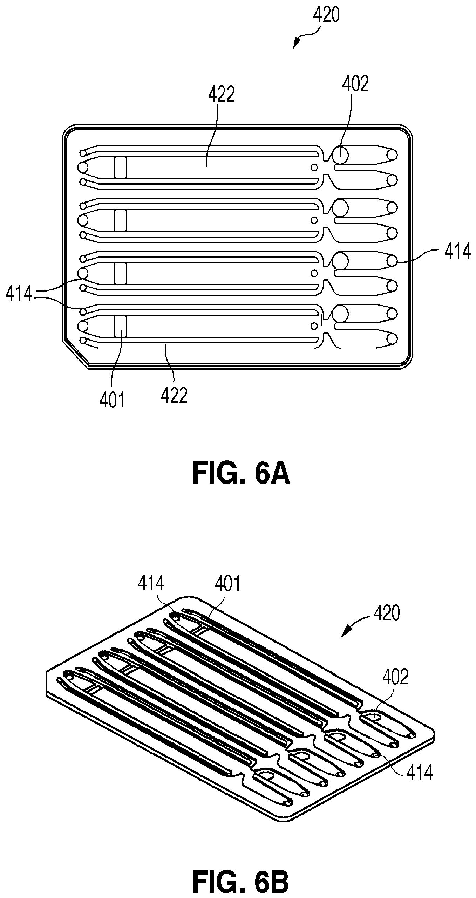

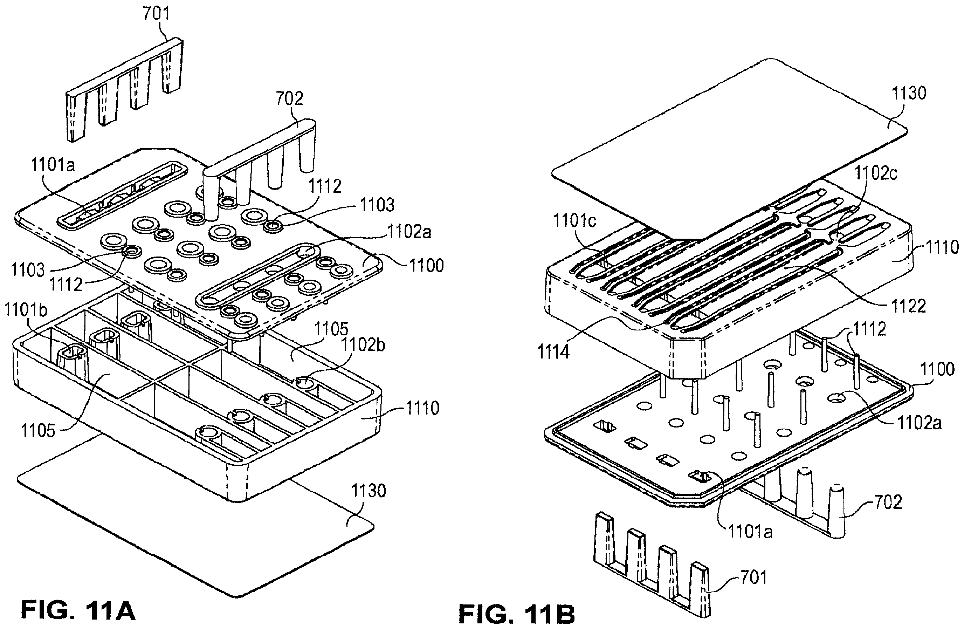

Bottom sheet **130** provides a closed bottom surface for channels **122** in channel sheet **120,** resulting in the channels being disposed within the card. Alternatively, channel sheet **120** and top sheet **110** may be combined in a single shaped layer that includes channels **122** etched, molded, or otherwise formed into the single layer, as seen, for example, in **FIGS. 6A** and **6B** and described below. In this alternative, the shaped layer will also include electrodes, vias, and wells. In yet another alternative, channel sheet **120** and bottom sheet **130** may be combined in a single shaped layer that includes channels **122** etched, molded, or otherwise formed into the single layer. When viewed from the top, such a structure would appear similar to structure **100** as it is shown in **FIG. 2A****.** In still another alternative, shown in **FIG. 11B****,** the card structures may be formed into the caddy, so that the card consists of only a bottom sheet.

**FIG. 1B** shows multilayer card **100** assembled, with top sheet **110** aligned with channel sheet **120,** which is aligned with bottom sheet **130.** The three sheets are bonded together using methods known in the art. Channels **122** are shown in **FIG. 1B** as if seen through a translucent or transparent top sheet **110.** However, if optical detection is required, top sheet **110** may be opaque to prevent unwanted excitation and fluorescence of the liquid contained in the channels, and bottom sheet **130** may be transparent to permit detection. Various detection schemes may be employed with devices according to the present invention, and so the layers may be formed from whatever material is appropriate to either prevent excitation or permit detection. The same material or different materials may be employed in the various layers, with poly(methyl methacrylate) (PMMA) and cyclic olefin copolymer (COC) being just two possible materials.

**FIGS. 2A** and **2B** show exploded views of multilayer card **100** and a caddy **200,** with the card uppermost in **FIG. 2A** and the caddy uppermost in **FIG. 2B****.** In operation, the caddy would be uppermost as shown in **FIG. 2A****.** The configuration of reservoirs **205** in caddy **200** is determined by the configuration of vias **114** in top sheet **110,** which, as described above, is based on the configuration of the channel or channels **122** in channel sheet **120.** Thus, while one configuration of reservoirs, vias, and channels is illustrated in the figures, embodiments of the device admit variation in the number and configuration of the various structures to help improve operational efficiency. When used to carry out an electrophoretic separation, a card includes a minimum of one channel, two vias, and two electrodes, while the caddy includes a minimum of two reservoirs.

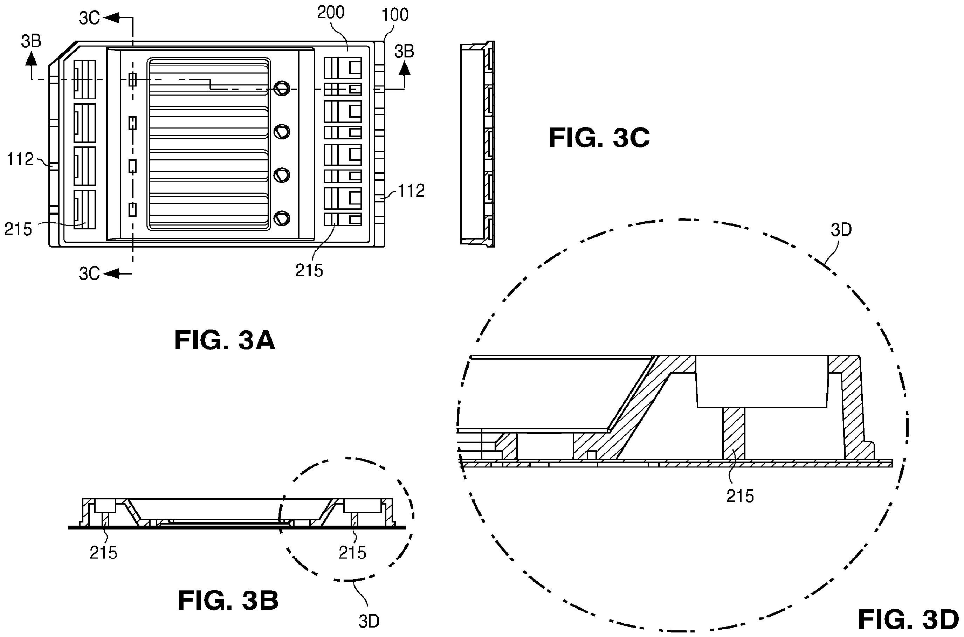

Caddy **200** is bonded to card **100** as seen in **FIG. 3A****,** resulting in a device having electrically isolated electrodes feeding into separated channels. Because card **100** is slightly larger than caddy **200,** a portion of each electrode extends beyond the caddy and is accessible at the edge of the card as seen in **FIG. 3A****.** As is evident from **FIGS. 2A** and **2B****,** when caddy **200** is mated with card **100,** each reservoir **205** overlaps both a portion of an electrode **112** and a via **114.** When a liquid is introduced into the reservoir, the liquid provides a conductive path between the electrode and a channel in fluid communication with the via. Thus, dry electrical contact can be made at the edge of the device that transfers current through the electrodes into the liquid-containing reservoirs. Therefore, an electrical contact that is included in an instrument designed to operate the device can be configured to contact only the portion of an electrode that extends beyond caddy **200** and not any liquid contained within the device, thereby avoiding cross-contamination between devices that are inserted into the instrument. As is evident from **FIGS. 1A** and **1B****,** wells **101** and **102** in the card are in fluid communication with channels **122.** Wells **201** and **202** in caddy **200** are aligned with wells **101** and **102** in the card as seen in **FIG. 2B****,** thereby permitting one or more samples to be introduced into the device through wells **201** and **101** and one or more isolated sample components to be removed from the device through wells **202** and **102.**

While it is presently preferred that the card be slightly larger than the caddy as described above, allowing a portion of each electrode to extend beyond the caddy, in alternative embodiments, the caddy and the card could be the same size or the caddy could even be larger than the card. In these alternatives, the portions of the electrodes disposed outside the reservoirs could be accessible through openings formed in the caddy or indentations in the edges of the caddy.

**FIGS. 3B** and **3D** show additional structures **215** that can be included in the reservoirs **205** of caddy **200.** These structures can prevent a separation matrix contained within a channel from flowing onto an electrode while allowing electrical contact by means of a buffer that touches both the separation matrix and the electrode.

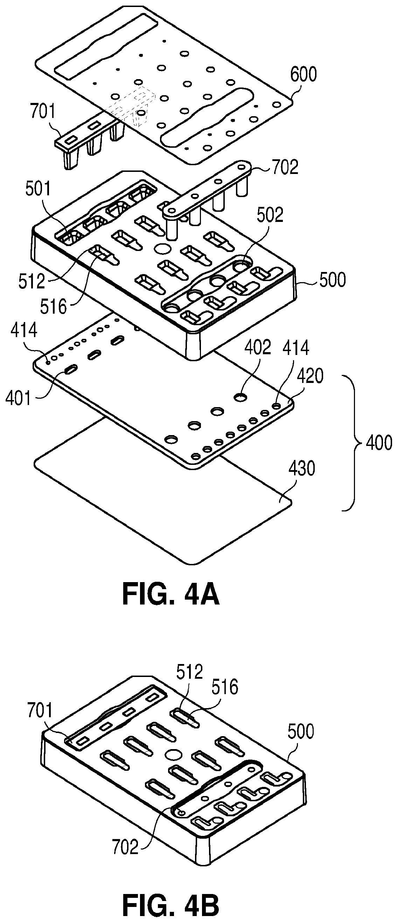

Another embodiment of a fluidic device having incorporated electrodes, in accordance with the present disclosure, is illustrated in **FIGS. 4A-8B****.** The illustrated device comprises a multilayer fluidic card **400** and a caddy **500.** In this embodiment, the electrodes are incorporated into the caddy rather than into the fluidic card. The multilayer card comprises a channel sheet **420** and a bottom sheet **430.** Channel sheet **420** includes wells **401** and **402,** vias **414,** and channels **422.** Electrodes **512** are disposed on deformable tabs **516** located on the top surface of caddy **500.** Caddy **500** also includes wells **501** and **502** and reservoirs **505.**

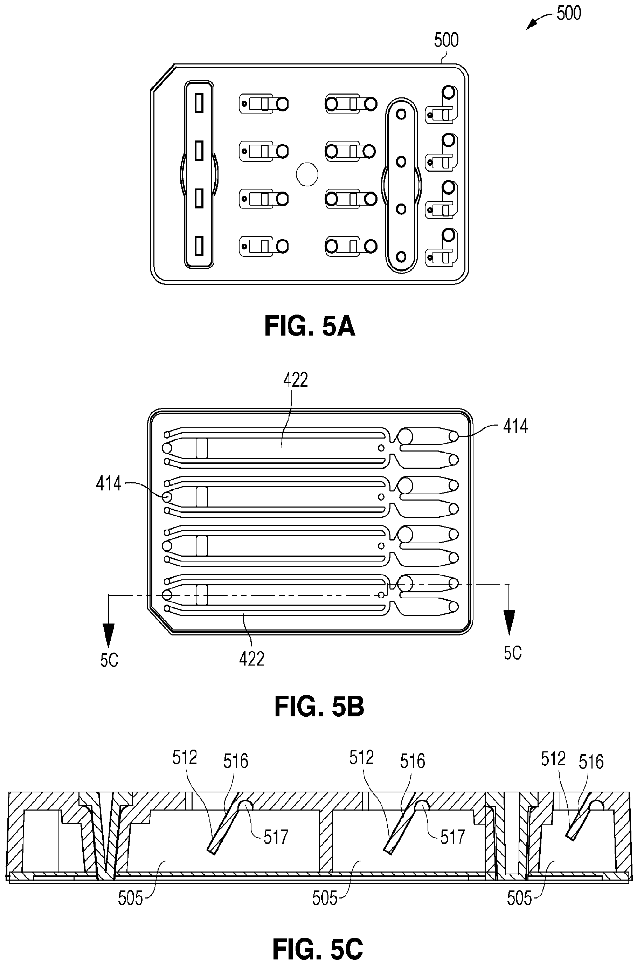

In the present embodiment, multilayer card **400** includes two layers: a shaped layer and a flat layer. The shaped layer, channel sheet **420,** is best seen in **FIGS. 6A** and **6B****.** **FIG. 4A** shows the top surface of channel sheet **420.** The bottom surface of channel sheet **420** would appear similar to the view seen in **FIG. 5B****.** In **FIG. 5B****,** which shows the bottom surface of the fluidic device, channels **422** are shown as if seen through a translucent or transparent bottom sheet **430.** Vias **414** pass entirely through channel sheet **420** and can be seen in all of **FIGS. 4A****,** **5B****,** **6A,** and **6B****.** Vias **414** may be formed using any appropriate technique known in the art, for example molding, etching, drilling, and laser cutting. In the present embodiment, channels **422** are machined into the bottom surface of sheet **420,** but they may be formed using other techniques, e.g., molding, etching, stamping, and grinding. As is best seen in **FIG. 6A****,** vias **414** provide fluid communication with channels **422.** Bottom sheet **430** provides a closed bottom surface for channels **422** in channel sheet **420.**

Alternative embodiments of multilayer card **400** are, of course, possible. For example, the multilayer card may include a top sheet, channel sheet, and bottom sheet, with vias and wells formed in the top sheet and channels formed in the channel sheet in the appropriate configurations, and the bottom sheet providing a closed bottom surface for the channels. Such a structure would appear similar to the multilayer fluidic card seen in **FIG. 1A** but without electrodes disposed on the surface of the card. In still another alternative, the card structures may be formed into the caddy, so that the card consists of only a bottom sheet.

Various detection schemes may be employed with devices according to the present embodiment, and so the layers of the multilayer fluidic card may be formed from whatever material is appropriate to either prevent excitation or permit detection. The same material or different materials may be employed in the various layers, with PMMA and COC being just two possible materials.

Caddy **500** includes both electrodes **512** and reservoirs **505.** Electrodes **512** are disposed on deformable tabs **516** to form electrode/tab structures on the top surface of caddy **500.** In the present embodiment, electrodes **512** are fabricated using a carbon conductive paste. In alternative embodiments, the electrodes may comprise different or additional materials, including, for example, gold, copper, platinum, silver/silver chloride paste, and other conductive materials. The electrodes may be patterned onto deformable tabs **516** by screen printing, pad printing, ink-jet printing, stenciling, or other similar methods. In addition, the electrodes may be formed on the tabs using a co-injection molding or an over-molding process. In alternative embodiments, electrodes **512** may be metal (or another conductive material) pieces bonded to caddy **500** or insert molded into the caddy. In these embodiments, electrodes **512** and deformable tabs **516** are integral structures rather than the electrodes being formed separately onto the deformable tabs.

The electrodes may comprise a single material or may comprise multiple materials. As in the previously described embodiment, multiple materials may be used where an electrode material has high conductivity but is incompatible with liquids to be used in and with the device. In this case, electrodes may be fabricated by applying a chemically compatible material (e.g., a carbon conductive paste) to regions of deformable tabs **516** that will come into contact with a liquid, applying a second material (e.g., a high-conductivity material such as silver) outside of the regions of the tabs that contact a liquid, and connecting the second material to the chemically compatible material. Alternatively, a chemically incompatible conductive material may be applied first to deformable tabs **516** and then covered completely with a chemically compatible conductive material. As a result, low electrical resistance is achieved, but any liquid within the device is exposed only to a chemically compatible material.

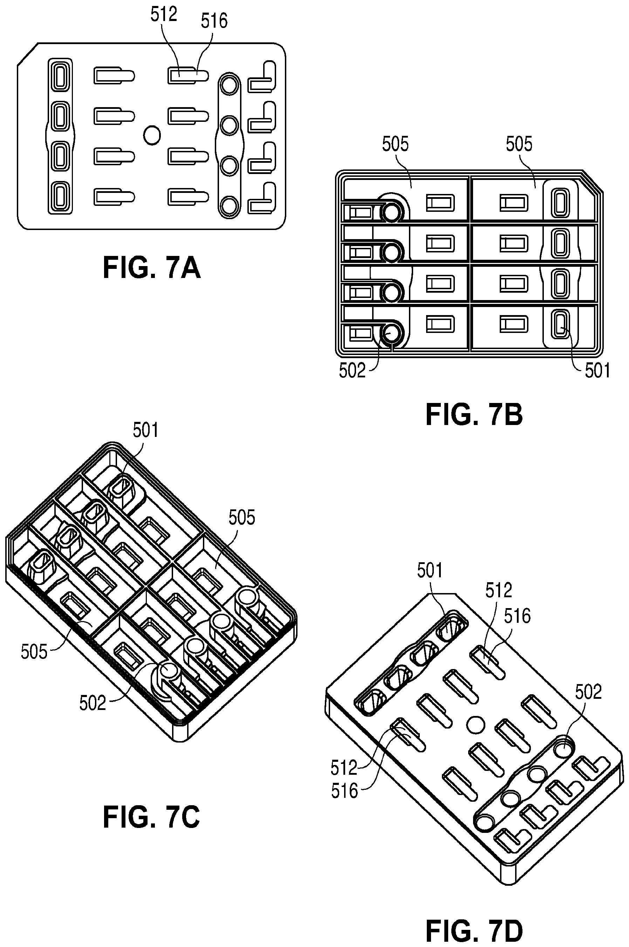

The deformable tabs are fabricated in a first position in the plane of the upper surface of the caddy. The tabs are deformed out of plane into a second position during a later stage of manufacture of the device. Thus, the term "deformable tab" is defined herein as a tab that is fabricated in a first (not deformed) position and that assumes a second (deformed) position during a later stage of manufacture of the device. In the second position, at least a portion of each tab **516** extends downward into one of reservoirs **505.** **FIGS. 7A** and **7D****,** for example, shows deformable tabs **516** in the first position, while **FIG. 5C** shows the tabs in the second position. Electrodes **512** may be formed on (or bonded to or molded into) the deformable tabs either before or after the tabs are deformed into the second position.

It may be advantageous to minimize the stress in the tab/electrode structure because the resistance of the electrode may increase when stretched or the electrode and/or tab may fracture when deformed. In one embodiment, it may be preferred to deform the tab/electrode structure in the shape of an arc, rather than at an angle as shown in **FIG. 5C****,** where the deformation of the tab/electrode structure is localized at a hinge point. Alternatively, or in addition, the tab/electrode structure could have multiple deformation points instead of a single one as shown in **FIG. 5C****.** The deformation point(s) may also be thinned to reduce stress. One means for thinning can be seen as semi-circular cutouts at **517** in **FIG. 5C****.**

Caddy **500** is bonded to card **400** as seen in **FIG. 4B** and **FIGS. 5A****-5C.** Either before or after the caddy and card are bonded together, each electrode/tab structure is deformed out of plane, as shown in **FIG. 5C****.** The electrode/tab structures are sized such that when reservoirs **505** contain a liquid, the electrodes reach down into the liquid. In this way, dry electrical contact can be made on the top of caddy **500** to transfer current into the liquid. Features (not shown) could be included on the top of the card or as part of the caddy that lock the electrode tabs in place after deforming.

While the figures show the contact areas for the dry electrodes spread out across the top of the device, the conductive traces could be routed to a single, more confined region of the device. This type of arrangement is often preferred for supporting a common electrical interface.

The configuration of reservoirs **505** in caddy **500** is partially determined by the configuration of vias **414** in channel sheet **420,** as when a liquid is introduced into a reservoir, the liquid provides a conductive path between the electrode **512** that reaches down into the liquid and a channel **422** through a via **414.** Liquid disposed in a first reservoir provides a conductive path between a first electrode and a first end of a channel through a first via; liquid in a second reservoir provides a conductive path between a second electrode and a second end of the channel through a second via. One configuration of reservoirs, vias, and channels is illustrated in the figures; however, embodiments of the device admit variation in the configuration of the various structures to help improve operational efficiency.

One particular advantage to the reservoir configuration of the present embodiment, which is best seen in **FIGS. 5C** and **FIG. 7C****,** is that when caddy **500** is joined to card **400,** reservoirs **505** are directly above channels **422,** in which electrophoresis may be carried out. In this configuration, liquid in the reservoirs positioned directly over the channels acts as a heat sink that can absorb joule heating during the electrophoresis process. In addition, this configuration is very compact and allows for a minimal total volume for the device. Reservoirs **505** are sized such that in combination they extend over an area covering substantially all of each of the channels of the device. This can be seen, for example, in **FIG. 5C****.** It will be appreciated that all of reservoirs **505** may be of equal size or may, alternatively, be of unequal size.

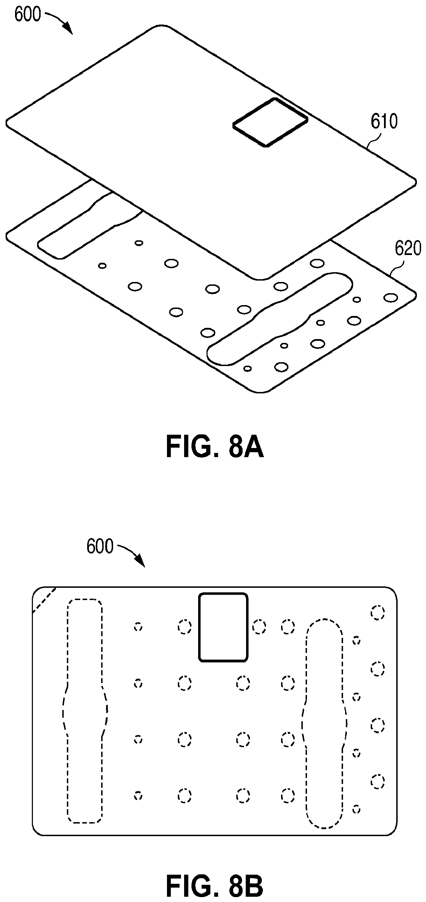

A patterned/punched label, seen at **600** in **FIGS. 4A****,** **8A,** and **8B****,** may be added on top of the caddy so that liquid within the device is completely sealed from the outside environment but openings are provided in the label to allow contact to the dry electrodes. As seen in **FIG. 8A****,** label **600** may comprise two layers, a cover sheet **610** and a patterned/punched sheet **620.** Cover sheet **610** is removed by the user of the device, leaving patterned/punched sheet **620** in place over caddy **500.**

Where a label is included in the device, the electrodes may be printed onto (or otherwise disposed on) the label rather than on the caddy. For example, electrode tabs could be incorporated into a flexible label as punch outs. In another embodiment, a laminate that includes molded plastic fingers could be applied on top of the caddy instead of being molded as part of the caddy. In either of these embodiments, the caddy itself would not be fabricated with deformable tabs, just openings above the reservoirs. Prior to use, the electrode tabs on the label would be punched and pushed into the reservoirs, or the molded plastic fingers would be bent so as to make electrical contact with a liquid in the caddy reservoirs. These alternative embodiments would not appear substantially different from the embodiment illustrated in the figures.

The device could also include blister packs for reagent storage. This would be desirable if some reagents are not stable when stored mixed in the device or otherwise need to be kept separate until point of use. The blister pack could be burst by the user or the instrument, releasing the stored reagent(s) into the reservoirs at the appropriate time.

Plugs (or combs) **701** and **702** can be seen in the figures. These are included to help ensure that wells **501** and **502** in caddy **500** and wells **401** and **402** in card **400** remain free of any separation matrix contained within the device during manufacturing, shipping, and storage. The plugs are removed prior to using the device, permitting one or more samples to be introduced into the device and one or more isolated sample components to be removed from the device through wells **501** and **502,** respectively, in the caddy, wells **501** and **502** being aligned with wells **401** and **402** in the card. The plugs also aid in removing any separation matrix that may have adhered to the walls of the wells. The unwanted separation matrix is dislodged from the well walls when the plugs are removed from the wells and drawn out along with the plugs. Preferably the plugs are manufactured from a porous material, for example a material thermally or laser sintered from a powder. Plug materials may include, but are not limited to, polyamide, polypropylene, high density polyethylene (HDPE), ethylene-vinyl acetate (EVA), and polystyrene (PS).

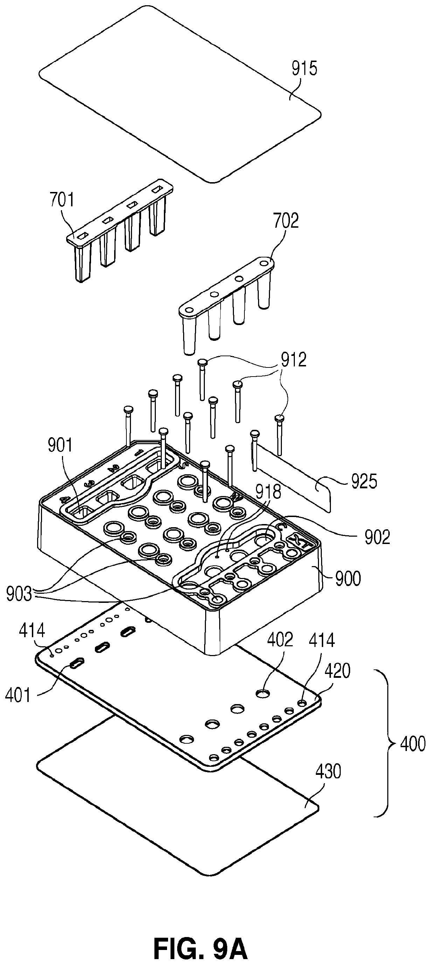

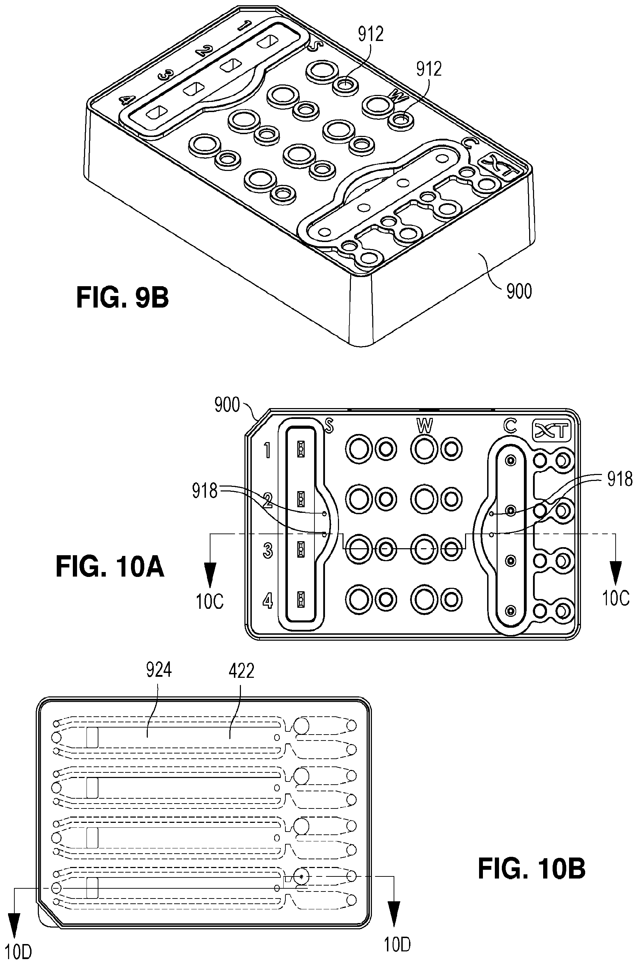

Yet another embodiment of a fluidic device having incorporated electrodes, in accordance with the present disclosure, is illustrated in **FIGS. 9A-10D****.** The illustrated device comprises a multilayer fluidic card and a caddy **900.** In this embodiment, as in the previous embodiment, the electrodes are incorporated into the caddy rather than into the fluidic card. As illustrated in **FIG. 9A****,** the multilayer card is card **400,** which is also illustrated in **FIG. 4A** and has been described above. Alternative multilayer cards have also been described above and may be used in place of the illustrated card **400.** A separation matrix **924** is disposed within at least a portion of one or more of the channels included in the multilayer card. Caddy **900** includes wells **901** and **902,** reservoirs **905,** and electrodes **912.** The device further includes a seal **915,** vents **918,** and a barcode label **925.**

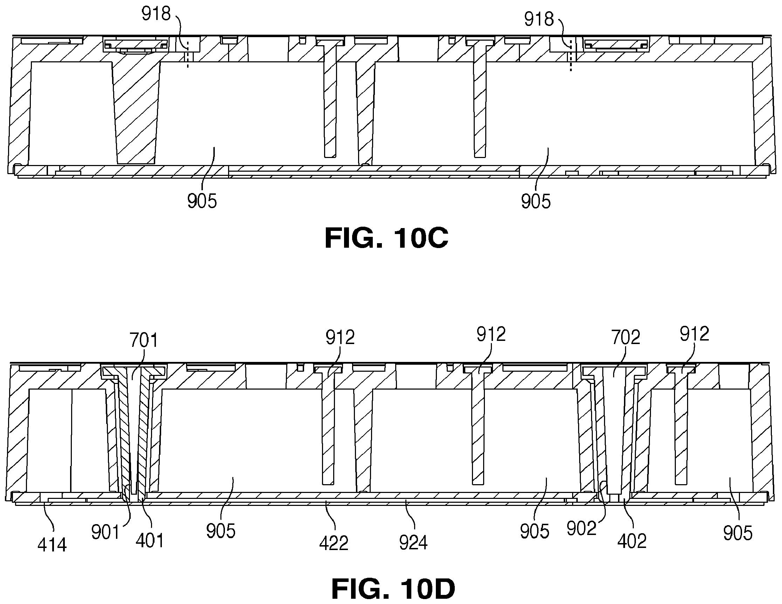

In the present embodiment, electrodes **912** are independent structures (i.e., structures that are fabricated separately from the caddy) that are incorporated into caddy **900** by being inserted (e.g., by being driven or press-fit) into the caddy. As seen in **FIG. 9A****,** openings **903** are provided in the caddy through which electrodes **912** extend. Electrodes **912** are shown in place within openings **903** in **FIG. 10D****.** While electrodes **912** are illustrated in the figures as straight metal pins, they may take shapes other than the one illustrated and may be fabricated from and/or coated with a conductive material other than a metal.

Electrodes **912** may be integrated into caddy **900** either before or after the caddy is bonded to card **400.** An upper portion of each electrode remains accessible on the upper surface of the device while a lower portion extends down into the reservoir over which the electrode is positioned. The electrodes are sized such that when reservoirs **905** contain a liquid, the electrodes extend down into the liquid. Thus, dry electrical contact can be made on the top of caddy **900** to transfer current into the liquid contained within reservoirs **905.** The liquid provides a conductive path between an electrode and a channel in fluid communication with a via in the fluidic card. Liquid disposed in a first reservoir provides a conductive path between a first electrode and a first end of a channel through a first via; liquid in a second reservoir provides a conductive path between a second electrode and a second end of the channel through a second via.

The configuration of reservoirs **905** in caddy **900** is partially determined by the configuration of the vias in the fluidic card. While one configuration of reservoirs, vias, and channels is illustrated in the figures, embodiments of the device admit variation in the configuration of the various structures to help improve operational efficiency. However, as noted above, one particular advantage to the reservoir configuration of both the present and the previous embodiment is that the reservoirs are directly above the channels. Where the channels are used for electrophoresis, liquid in the reservoirs positioned directly over the channels acts as a heat sink to absorb joule heating during the electrophoresis process. Reservoirs **905** are sized such that in combination they extend over an area covering substantially all of each of the channels of the device. This can be seen, for example, in **FIG. 10D****.** It will be appreciated that all of reservoirs **905** may be of equal size or may, alternatively, be of unequal size.

Seal **915** may be a solid sheet of material, such as a heat-seal polymer or foil, that is used to cover the device during shipping. Alternatively, a patterned/punched label such as is seen at **600** in **FIGS. 4A****,** **8A,** and **8B** may be used. Note that various raised areas are provided on the surface of caddy **900.** These are especially important when seal **915** comprises a heat seal and are used to concentrate heat and pressure near regions that must be sealed (e.g., wells) and to protect other regions from contact with the seal (e.g., the exposed upper surfaces of the electrodes). The features have narrow rims to reduce the force that a user must exert to peel off the seal before using the device. These features may be eliminated if a seal is not used with the device.

Vents **918** are included to protect a sealed device from damage during shipping and storage. If a device is subjected to conditions such as, for example, heat or reduced pressure while seal **915** is in place, the portion of seal **915** affixed over the area of a plug **701** or **702** may expand, creating a vacuum within the plug area. If the vacuum is not otherwise relieved, it can extend beneath the plug and through one or more of caddy wells **901** or **902** into the associated well(s) **401** and **402** within the fluidic card. As can be best seen in **FIGS. 9A** and **10D****,** each of wells **401** and **402** within the fluidic card is fluidly connected with a channel **422.** A vacuum that acts on any of these wells can draw a separation matrix **924** disposed within the channel **422** out of the channel and into the well, affecting the utility of the device. To eliminate the possibility of a vacuum being communicated from a plug area to a channel through one or more wells, vents **918** are positioned adjacent to the wells as seen, for example, in **FIGS. 9A****,** **10A****,** and **10C****.** Referring now to **FIG. 10C****,** it can be seen that vents **918** connect each plug area with a reservoir **905** below. By connecting the plug area to the reservoir, which is only partially filled with a fluid (e.g., a buffer), any vacuum created during shipping or storage is transferred to the air space over the fluid within the reservoir. Because the volume of the reservoir is much larger than the extra volume created by the expansion of the portion of the seal affixed over the plug area, the vacuum created by the seal expansion is damped out (by the ratio of the volumes). Additionally, each vent **918** creates a pressure short circuit between a well and a via as can be seen, for example, between well **401** and via **414** in **FIG. 10D****.** Therefore, even if there is some residual pressure change due to expansion of the seal, there is no pressure difference between the channel and the well, and the separation matrix does not flow out of the channel.

In the present embodiment, label **925** includes a 2D barcode. The barcode may encode, for example, lot- or batch-specific information for the device.

An embodiment of a fluidic device having incorporated electrodes, in accordance with the present invention, is illustrated in **FIGS. 11A** and **11B****.** This embodiment is similar to the embodiment described immediately above and illustrated in **FIGS. 9A-10D****;** however, in the present embodiment, the card structures (i.e., those structures found in a fluidic card in the embodiments described above) are formed into the caddy, with the card **1130** consisting of only a bottom sheet. To facilitate forming the card structures into the caddy, the caddy comprises two segments: a first segment **1100,** also referred to herein as a top segment, and a second segment **1110,** also referred to herein as a bottom segment. Electrodes **1112** are incorporated into caddy segment **1100.** The device may further comprise any of the features previously described (e.g., vents such as have been described above and illustrated at **918** in **FIG. 9A** and/or a barcode label such as has been described above and illustrated at **925** in **FIG. 9A**).

As seen in **FIGS. 11A** and **11B****,** the caddy includes wells that extend through both of the caddy segments, with first well portions **1101a** and **1102a** disposed in caddy top segment **1100,** second portions **1110b** and **1102b** disposed in caddy bottom segment **1110,** and third portions **1110c** and **1102c** also disposed in caddy bottom segment **1110.** When caddy top segment **1100** is attached to caddy bottom segment **1110,** well portions **1101a, 1101b,** and **1101c** are in fluid communication, and well portions **1102a, 1102b,** and **1102c** are in fluid communication. When the caddy segments are assembled, the well portions combine to form wells that are in fluid communication with channels **1122.**

Caddy bottom segment **1110** includes a shaped top surface, seen in **FIG. 11A****,** that includes reservoirs **1105,** and a shaped bottom surface, seen in **FIG. 11B****,** that includes well portions, as described above, and channels **1122.** Vias **1114** extend through caddy bottom segment **1110** from the top surface to the bottom surface and provide fluid communication between reservoirs **1105** and channels **1122.** For example, a first via provides fluid communication between a first reservoir and a first end of a channel, and a second via provides fluid communication between a second reservoir and a second end of the channel. Card **1130** is attached to caddy bottom segment **1110** to provide a closed bottom surface for the device, resulting in the structures seen in **FIG 11B** being disposed within the device.

Reservoirs **1105** are sized such that in combination they extend over an area that covers substantially all of each of the channels of the device. As is evident from **FIGS. 11A** and **11B****,** a combination of a first and a second reservoir extends over an area covering substantially all of each channel of the device. Where channels **1122** are used for electrophoresis, liquid in reservoirs**1105** acts as a heat sink to absorb joule heating during the electrophoresis process. Reservoirs **1105** may be of equal or unequal sizes.

In the present embodiment, electrodes **1112** are independent structures (i.e., structures that are fabricated separately from the caddy) that are incorporated into caddy top segment **1100** by being inserted (e.g., by being driven or press-fit) into the caddy. As seen in **FIG. 11A****,** openings **1103** are provided in caddy top segment **1100** through which electrodes **1112** extend. In **FIGS. 11A** and **11B****,** electrodes **1112** are shown in place within openings **1103.** While electrodes **1112** are illustrated in the figures as substantially straight metal pins, they may take shapes other than the one illustrated. For example, electrodes **1112** may be disposed on deformable tabs according to any of the variations described above. Electrodes **1112** may be fabricated from and/or coated with a conductive material other than a metal.

Electrodes **1112** may be integrated into caddy top segment **1100** either before or after caddy top segment **1100** is attached to caddy bottom segment **1110** to form a single caddy structure. The top and bottom caddy segments may be attached one to the other by means of an adhesive, a snap-fit design, or other means known in the art. An upper portion of each electrode (a first portion) remains accessible on the upper surface of the device for dry electrical contact, while a lower portion (a second portion) of each electrode extends down into the reservoir **1105** over which the electrode is positioned. The electrodes are sized such that when the reservoirs contain a liquid, the electrodes extend down into the liquid. Thus, dry electrical contact can be made on the top of the device to transfer current into the liquid contained within reservoirs **1105.** The liquid provides a conductive path between an electrode **1112** and a channel **1122** that is in fluid communication with a reservoir **1105** through a via **1114.** Liquid disposed in a first reservoir provides a conductive path between a first electrode and a first end of a channel through a first via; liquid in a second reservoir provides a conductive path between a second electrode and a second end of the channel through a second via. The number of channels in the device may vary from the number illustrated in **FIG. 11B****.**

It should be noted that a caddy similar to those seen in **FIGS. 5C****,** **7C****,** **10C, 10D****,** **11A,** and **11B** (i.e., a caddy having reservoirs that extend over the area occupied by channels and/or chambers in a fluidic device, thereby acting as a heat sink for the channels and/or chambers) will offer advantages in any number of fluidic devices, not only fluidic devices incorporating electrodes, but also fluidic devices that are operated using electrodes located separate from the device in a system designed to receive the fluidic device.

Thus, also disclosed herein is a fluidic device configured to absorb joule heating within the device, the device comprising a card and a caddy. Such a device would be, in effect, a device such as those that have been described above but without the electrodes. The card may be any of the cards described above and illustrated in the figures. The card comprises a channel having first and second ends, the channel disposed within the card. The card further comprises first and second vias in fluid communication with the channel through an upper surface of the card. The first via is positioned adjacent to the first end of the channel, and the second via is positioned adjacent to the second end of the channel. The caddy comprises at least first and second reservoirs and may appear similar to the caddy seen in **FIG. 7C****.** The caddy is attached to the card such that the first reservoir is positioned over the first via and the second reservoir is positioned over the second via. The first and second reservoirs are sized such that the two reservoirs in combination extend over an area covering substantially all of the channel disposed within the card.

Another aspect of the present invention is a system for isolating one or more sample components of a sample material following separation of the sample material into a plurality of sample components. The system comprises a device such as those that have been described above as well as instrumentation for controlling the device. For example, the instrumentation may comprise a detector positioned in sensory communication with a detection region of the device, a processor operably coupled to the detector and to a fluid direction system that is configured to control movement of one or more sample components based on information received from the detector. As used herein, the phrase "in sensory communication" refers to positioning of a detector such that it is operably connected to the device, i.e., capable of receiving a detectable signal from the contents of the device. In the case of optical signals, this requires only that the detector be positioned to receive the optical signal. The system may be configured to simultaneously control multiple channels or multiple fluidic circuits. In such a configuration, the fluid direction system may be configured to control the movement of one or more sample components in one channel or fluidic circuit based on information received by the detector in a parallel channel or circuit. Fluid movement may be controlled by the use of electrokinetics, such as electrophoresis or electroosmosis, wherein charged components within the fluid move, and/or the fluid itself moves, in response to the application of positive and negative voltages. Another example includes the application of positive or negative partial pressure to certain areas of the device, so that fluid moves from locations of high pressure to locations of low pressure through the channel or channels of the device. Partial pressures may be applied by a pumping mechanism or the application of a vacuum force to a reservoir. Therefore, the fluid direction system includes the capabilities for providing and controlling forces to move fluids or fluid components through the channel or channels of the device.

Another system for isolating one or more sample components of a sample material following separation of the sample material into a plurality of sample components comprises a fluidic device having incorporated electrodes, a detector in sensory communication with the device, a fluid direction system, and a processor operably coupled to the detector and the fluid direction system. The device comprises a card, a first caddy segment, and a second caddy segment. The first caddy segment comprises first and second electrodes. The second caddy segment comprises first and second reservoirs, a channel, and first and second vias. The reservoirs are disposed on a first surface of the second segment. The channel is disposed on a second surface of the second segment. The vias extend between the first and second surfaces, the first via providing fluid communication between the first reservoir and a first end of the channel, and the second via providing fluid communication between the second reservoir and a second end of the channel.

Also disclosed herein is a method for manufacturing a fluidic device having incorporated electrodes. In a first method, a card and a caddy are provided. The card comprises a channel, first and second vias, and first and second electrodes. The channel is disposed within the card and has first and second ends. The first and second vias are in fluid communication with the channel through an upper surface of the card. The first and second electrodes are disposed on the upper surface of the card. The first via and first electrode are positioned adjacent to the first end of the channel, and the second via and second electrode are positioned adjacent to the second end of the channel.

The caddy includes first and second reservoirs. The caddy is attached to the card such that the first reservoir is positioned over the first via and a portion of the first electrode, the second reservoir is positioned over the second via and a portion of the second electrode, and a portion of each of the first electrode and the second electrode is accessible for dry electrical contact.

In a second method for manufacturing fluidic devices having incorporated electrodes, a card and a caddy are provided. The card comprises a channel and first and second vias. The channel is disposed within the card and has first and second ends. The first via is in fluid communication with the channel through an upper surface of the card and is positioned adjacent to the first end of the channel. The second via is in fluid communication with the channel through the upper surface of the card and is positioned adjacent to the second end of the channel.

The caddy comprises first and second reservoirs and first and second electrodes. The first electrode is positioned over the first reservoir, and the second electrode is positioned over the second reservoir. In one embodiment of the caddy, the first electrode is disposed on a first deformable tab, and the second electrode is disposed on a second deformable tab. The tabs are formed on the caddy in a first position and are then deformed out of plane into a second position. The electrodes may be formed on the tabs either before or after the tabs are deformed, and the tabs may be deformed either before or after the caddy is attached to the card. In a second embodiment of the caddy, the first and second electrodes are independent structures (e.g., metal pins) that are incorporated into the caddy by being inserted (driven or press-fit) into openings provided in the caddy. In this embodiment, the electrodes may be incorporated into the caddy either before or after the caddy is attached to the card.

The caddy is attached to the card such that the first reservoir is positioned over the first via and the second reservoir is positioned over the second via and such that a portion of each of the first electrode and the second electrode is accessible for dry electrical contact.

While the embodiments of the invention disclosed herein are presently considered to be preferred, various changes and modifications can be made without departing from the scope of the invention. The scope of the invention is indicated in the appended claims.

## Claims

1. A fluidic device having incorporated electrodes, comprising:
a card (1130); and
a caddy, the caddy comprising:
a first caddy segment (1100); and
a second caddy segment (1110) comprising a first surface and a second surface;
wherein the card (1130) is attached to the second surface of the second caddy segment such that the card provides a closed surface for the device;
**characterized in that:**
the first caddy segment (1100) comprises first and second electrodes (1112);
the second caddy segment (1110) comprises first and second reservoirs (1105) disposed on the first surface of the second caddy segment, a channel (1122) disposed on the second surface of the second caddy segment, and first and second vias (1114) extending between the first and second surfaces to provide fluid communication between the reservoir (1105) and the channel (1122); and
the first caddy segment (1100) is attachable to the first surface of the second caddy segment (1110) such that, when attached, a first portion of the first electrode (1112) is accessible for dry electrical contact and a second portion of the first electrode extends into the first reservoir (1105) and such that a first portion of the second electrode is accessible for dry electrical contact and a second portion of the second electrode extends into the second reservoir (1105).

2. The device of claim 1 wherein the card (1130) is a bottom sheet that provides the closed surface for the device.

3. The device of claim 1 wherein the first via (1114) provides fluid communication between the first reservoir (1105) and a first end of the channel, and the second via (1114) provides fluid communication between the second reservoir (1105) and a second end of the channel.

4. The device of claim 1 further comprising a liquid disposed in each of the first and second reservoirs (1105), wherein the liquid disposed in the first reservoir (1105) provides a conductive path between the first electrode (1112) and the first end of the channel through the first via (1114), and wherein the liquid disposed in the second reservoir (1105) provides a conductive path between the second electrode and the second end of the channel through the second via (1114).

5. The device of claim 1 wherein the caddy further comprises first and second wells in fluid communication with the channel (1122).

6. The device of claim 1 wherein the first and second reservoirs (1105) are sized such that the reservoirs (1105) in combination extend over an area covering substantially all of the channel (1122).

7. The device of claim 1 wherein the second caddy segment (1110) comprises a plurality of channels (1122), a plurality of first and second reservoirs (1105), and a plurality of first and second vias (1114), optionally wherein each combination of a first and a second reservoir (1105) is sized such that the combination extends over an area covering substantially all of at least one of the plurality of channels (1122).

8. The device of claim 6 wherein:
(a) each channel (1122) has a first end and a second end and wherein a first via (1114) provides fluid communication between the first end of each channel and a first reservoir (1105) and a second via (1114) provides fluid communication between the second end of each channel and a second reservoir (1105); or
(b) for each of the plurality of channels (1122), a first via (1114) provides fluid communication between a first reservoir (1105) and a first end of the channel, and a second via (1114) provides fluid communication between a second reservoir (1105) and a second end of the channel.

9. The device of claim 1 wherein the first and second electrodes (1112) are fabricated as structures independent of the caddy and are inserted into openings in the first caddy segment (1100).

10. The device of claim 1 wherein the first electrode (1112) is disposed on a first deformable tab and the second electrode (1112) is disposed on a second deformable tab.

11. The device of claim 10 wherein the deformable tabs are formed into the first caddy segment (1100) or the deformable tabs are attached to a surface of the first caddy segment.

12. The device of claim 10 further comprising a label attached to a surface of the first caddy segment.

13. The device of claim 12 wherein the deformable tabs are formed into the label or the deformable tabs are attached to the label.

14. The device of claim 10 wherein the deformable tabs are deformed during manufacture such that, when the first caddy segment (1100) is attached to the first surface of the second caddy segment (1110), a portion of the first electrode (1112) extends downward into the first reservoir (1105) and a portion of the second electrode (1112) extends downward into the second reservoir (1105).

15. The fluidic device of claim 5, the device further comprising:
first and second vents disposed in the caddy adjacent to the first and second wells, the first vent in fluid communication with the first reservoir (1105) and the second vent in fluid communication with the second reservoir (1105), wherein the vents equilibrate pressure differences that result when a seal that is affixed over the first and second wells expands, creating a vacuum beneath the seal.

16. A system for isolating one or more sample components of a sample material following separation of the sample material into a plurality of sample components, the system comprising:
a fluidic device having incorporated electrodes, comprising:
a card (1130); and
a caddy, the caddy comprising:
a first caddy segment (1100); and
a second caddy segment (1110) comprising a first surface and a second surface;
wherein the card (1130) is attached to the second surface of the second caddy segment such that the card provides a closed surface for the device; and
a detector in sensory communication with the device;
a fluid direction system; and
a processor operably coupled to the detector and the fluid direction system;
**characterized in that:**
the first caddy segment (1100) comprises first and second electrodes (1112);
the second caddy segment (1110) comprises first and second reservoirs (1105) disposed on the first surface of the second caddy segment, a channel (1122) disposed on the second surface of the second caddy segment, and first and second vias (1114) extending between the first and second surfaces to provide fluid communication between the reservoir (1105) and the channel (1122); and
the first caddy segment (1100) is attachable to the first surface of the second caddy segment (1110) such that, when attached, a first portion of the first electrode (1112) is accessible for dry electrical contact and a second portion of the first electrode extends into the first reservoir (1105) and such that a first portion of the second electrode is accessible for dry electrical contact and a second portion of the second electrode extends into the second reservoir (1105).

## Patentansprüche

1. Fluidische Vorrichtung mit darin eingesetzten Elektroden, umfassend:
eine Karte (1130); und
einen Behälter, wobei der Behälter Folgendes umfasst:
ein erstes Behältersegment (1100); und
ein zweites Behältersegment (1110), das eine erste Oberfläche und eine zweite Oberfläche umfasst;
wobei die Karte (1130) an der zweiten Oberfläche des zweiten Behältersegments angebracht ist, derart, dass die Karte für die Vorrichtung eine geschlossene Oberfläche bereitstellt;
**dadurch gekennzeichnet,**
**dass** das erste Behältersegment (1100) eine erste und eine zweite Elektrode (1112) umfasst;
**dass** das zweite Behältersegment (1110) ein erstes und ein zweites Reservoir (1105), die auf der ersten Oberfläche des zweiten Behältersegments angeordnet sind, einen Kanal (1122), der auf der zweiten Oberfläche des zweiten Behältersegments angeordnet ist, und erste und zweite Durchgangslöcher (1114), die sich zwischen der ersten und der zweiten Oberfläche erstrecken, um Fluidkommunikation zwischen dem Reservoir (1105) und dem Kanal (1122) bereitzustellen, umfasst; und
**dass** das erste Behältersegment (1100) an der ersten Oberfläche des zweiten Behältersegments (1110) anbringbar ist, derart, dass bei Anbringung ein erster Abschnitt der ersten Elektrode (1112) für elektrischen Trockenkontakt zugänglich ist und ein zweiter Abschnitt der ersten Elektrode sich in das erste Reservoir (1105) hinein erstreckt, derart, dass ein erster Abschnitt der zweiten Elektrode für elektrischen Trockenkontakt zugänglich ist und ein zweiter Abschnitt der zweiten Elektrode sich in das zweite Reservoir (1105) hinein erstreckt.

2. Vorrichtung nach Anspruch 1, wobei die Karte (1130) eine Unterseitenplatte ist, die für die Vorrichtung die geschlossene Oberfläche bereitstellt.

3. Vorrichtung nach Anspruch 1, wobei das erste Durchgangsloch (1114) Fluidkommunikation zwischen dem ersten Reservoir (1105) und einem ersten Ende des Kanals bereitstellt, und das zweite Durchgangsloch (1114) Fluidkommunikation zwischen dem zweiten Reservoir (1105) und einem zweiten Ende des Kanals bereitstellt.

4. Vorrichtung nach Anspruch 1, ferner umfassend eine Flüssigkeit, die in jedem von dem ersten und dem zweiten Reservoir (1105) angeordnet ist, wobei die in dem ersten Reservoir (1105) angeordnete Flüssigkeit einen leitenden Pfad zwischen der ersten Elektrode (1112) und dem ersten Ende des Kanals durch das erste Durchgangsloch (1114) hindurch bereitstellt, und wobei die in dem zweiten Reservoir (1105) angeordnete Flüssigkeit einen leitenden Pfad zwischen der zweiten Elektrode und dem zweiten Ende des Kanals durch das zweite Durchgangsloch (1114) hindurch bereitstellt.

5. Vorrichtung nach Anspruch 1, wobei der Behälter ferner eine erste und eine zweite Vertiefung umfasst, die in Fluidkommunikation mit dem Kanal (1122) sind.

6. Vorrichtung nach Anspruch 1, wobei das erste und das zweite Reservoir (1105) derart bemessen sind, dass sich die Reservoirs (1105) in Kombination über eine Fläche erstrecken, die im Wesentlichen den gesamten Kanal (1122) abdeckt.

7. Vorrichtung nach Anspruch 1, wobei das zweite Behältersegment (1110) eine Vielzahl von Kanälen (1122), eine Vielzahl von ersten und zweiten Reservoirs (1105) und eine Vielzahl von ersten und zweiten Durchgangslöchern (1114) umfasst, wobei gegebenenfalls jede Kombination eines ersten und eines zweiten Reservoirs (1105) derart bemessen ist, dass sich die Kombination über eine Fläche erstreckt, die im Wesentlichen die Gesamtheit von zumindest einem aus der Vielzahl von Kanälen (1122) abdeckt.

8. Vorrichtung nach Anspruch 6,
wobei (a) jeder Kanal (1122) ein erstes Ende und ein zweites Ende aufweist, und wobei ein erstes Durchgangsloch (1114) Fluidkommunikation zwischen dem ersten Ende von jedem Kanal und einem ersten Reservoir (1105) bereitstellt, und ein zweites Durchgangsloch (1114) Fluidkommunikation zwischen dem zweiten Ende von jedem Kanal und einem zweiten Reservoir (1105) bereitstellt; oder
wobei (b) für jeden aus der Vielzahl von Kanälen (1122) ein erstes Durchgangsloch (1114) Fluidkommunikation zwischen einem ersten Reservoir (1105) und einem ersten Ende des Kanals bereitstellt, und ein zweites Durchgangsloch (1114) Fluidkommunikation zwischen einem zweiten Reservoir (1105) und einem zweiten Ende des Kanals bereitstellt.

9. Vorrichtung nach Anspruch 1, wobei die erste und die zweite Elektrode (1112) als Strukturen hergestellt sind, die von dem Behälter unabhängig sind, und in Öffnungen im ersten Behältersegment (1100) eingebracht sind.

10. Vorrichtung nach Anspruch 1, wobei die erste Elektrode (1112) auf einer ersten verformbaren Nase angeordnet ist und die zweite Elektrode (1112) auf einer zweiten verformbaren Nase angeordnet ist.

11. Vorrichtung nach Anspruch 10,
wobei die verformbaren Nasen im ersten Behältersegment (1100) ausgebildet sind oder die verformbaren Nasen an einer Oberfläche des ersten Behältersegments angebracht sind.

12. Vorrichtung nach Anspruch 10,
ferner umfassend eine Kennzeichnung, die an einer Oberfläche des ersten Behältersegments angebracht ist.

13. Vorrichtung nach Anspruch 12,
wobei die verformbaren Nasen in der Kennzeichnung ausgebildet sind oder die verformbaren Nasen an der Kennzeichnung angebracht sind.

14. Vorrichtung nach Anspruch 10,
wobei die verformbaren Nasen während der Herstellung derart verformt werden, dass, wenn das erste Behältersegment (1100) an der ersten Oberfläche des zweiten Behältersegments (1110) angebracht wird, ein Abschnitt der ersten Elektrode (1112) sich nach unten, in das erste Reservoir (1105) hinein erstreckt, und ein Abschnitt der zweiten Elektrode (1112) sich nach unten, in das zweite Reservoir (1105) hinein erstreckt.

15. Fluidische Vorrichtung nach Anspruch 5,
wobei die Vorrichtung ferner Folgendes umfasst:
eine erste und eine zweite Entlüftungsbohrung, die in dem Behälter neben der ersten und der zweiten Vertiefung angeordnet sind, wobei die erste Entlüftungsbohrung in Fluidkommunikation mit dem ersten Reservoir (1105) ist, und die zweite Entlüftungsbohrung in Fluidkommunikation mit dem zweiten Reservoir (1105) ist, wobei die Entlüftungsbohrungen Druckunterschiede ausgleichen, die sich beim Ausdehnen einer Dichtung, die über der ersten und der zweiten Vertiefung befestigt ist, ergeben, wodurch unterhalb der Dichtung ein Vakuum entsteht.

16. System zur Isolierung von einer oder mehreren Komponenten eines Probenmaterials nach dem Trennen des Probenmaterials in eine Vielzahl von Probenkomponenten, wobei das System Folgendes umfasst:
eine fluidische Vorrichtung mit darin eingesetzten Elektroden, umfassend:
eine Karte (1130); und
einen Behälter, wobei der Behälter Folgendes umfasst:
ein erstes Behältersegment (1100); und
ein zweites Behältersegment (1110), das eine erste Oberfläche und eine zweite Oberfläche umfasst;
wobei die Karte (1130) an der zweiten Oberfläche des zweiten Behältersegments angebracht ist, derart, dass die Karte für die Vorrichtung eine geschlossene Oberfläche bereitstellt; und
einen Detektor, der mit der Vorrichtung in sensorischer Kommunikation ist;
ein Fluidlenkungssystem; und
einen Prozessor, der mit dem Detektor und dem Fluidlenkungssystem wirk-gekoppelt ist,
**dadurch gekennzeichnet,**
**dass** das erste Behältersegment (1100) eine erste und eine zweite Elektrode (1112) umfasst;
**dass** das zweite Behältersegment (1110) das erste und das zweite Reservoir (1105), die auf der ersten Oberfläche des zweiten Behältersegments angeordnet sind, einen Kanal (1122), der auf der zweiten Oberfläche des zweiten Behältersegments angeordnet ist, und erste und zweite Durchgangslöcher (1114), die sich zwischen der ersten und der zweiten Oberfläche erstrecken, um Fluidkommunikation zwischen dem Reservoir (1105) und dem Kanal (1122) bereitzustellen, umfasst; und
**dass** das erste Behältersegment (1100) an der ersten Oberfläche des zweiten Behältersegments (1110) anbringbar ist, derart, dass bei Anbringung ein erster Abschnitt der ersten Elektrode (1112) für elektrischen Trockenkontakt zugänglich ist und ein zweiter Abschnitt der ersten Elektrode sich in das erste Reservoir (1105) hinein erstreckt, derart, dass ein erster Abschnitt der zweiten Elektrode für elektrischen Trockenkontakt zugänglich ist und ein zweiter Abschnitt der zweiten Elektrode sich in das zweite Reservoir (1105) hinein erstreckt.

## Revendications

1. Dispositif fluidique comportant des électrodes incorporées, comprenant :
une carte (1130) ; et
un caddie, le caddie comprenant :
un premier segment de caddie (1100) ; et
un second segment de chariot (1110) comprenant une première surface et une seconde surface ;
dans lequel la carte (1130) est fixée à la seconde surface du second segment de caddie de telle sorte que la carte fournisse une surface fermée pour le dispositif ;
**caractérisé en ce que** :
le premier segment de caddy (1100) comprend des première et seconde électrodes (1112) ;
le second segment de caddie (1110) comprend des premier et second réservoirs (1105) disposés sur la première surface du deuxième segment de caddie, un canal (1122) disposé sur la seconde surface du second segment de caddie et des premier et second vias (1114)
s'étendant entre les première et seconde surfaces pour établir une communication de fluide entre le réservoir (1105) et le canal (1122) ; et
le premier segment de chariot (1100) peut être fixé à la première surface du second segment de chariot (1110) de telle sorte que, une fois fixée, une première partie de la première électrode (1112) est accessible pour le contact électrique sec et une seconde partie du premier l'électrode s'étend dans le premier réservoir (1105) et de telle sorte qu'une première partie de la deuxième électrode est accessible pour un contact électrique sec et qu'une deuxième partie de la deuxième électrode s'étend dans le deuxième réservoir (1105).

2. Dispositif selon la revendication 1, dans lequel la carte (1130) est une feuille inférieure qui fournit la surface fermée pour le dispositif.

3. Dispositif selon la revendication 1, dans lequel le premier via (1114) établit une communication fluidique entre le premier réservoir (1105) et une première extrémité du canal, et le second via (1114) assure la communication fluidique entre le second réservoir (1105) et une deuxième extrémité du canal.

4. Dispositif selon la revendication 1, comprenant en outre un liquide disposé dans chacun des premier et second réservoirs (1105), le liquide disposé dans le premier réservoir (1105) constituant un chemin conducteur entre la première électrode (1112) et la première extrémité du canal à travers le premier trou d'interconnexion (1114), et dans lequel le liquide disposé dans le deuxième réservoir (1105) constitue un chemin conducteur entre la deuxième électrode et la deuxième extrémité du canal à travers le deuxième trou d'interconnexion (1114).

5. Dispositif selon la revendication 1, dans lequel le chariot comprend en outre des premier et second puits en communication de fluide avec le canal (1122).

6. Dispositif selon la revendication 1, dans lequel les premier et second réservoirs (1105) sont dimensionnés de sorte que les réservoirs (1105) en combinaison s'étendent sur une zone couvrant sensiblement la totalité du canal (1122).

7. Dispositif selon la revendication 1, dans lequel le deuxième segment de chariot (1110) comprend une pluralité de canaux (1122), une pluralité de premier et second réservoirs (1105) et une pluralité de premier et second vias (1114), dans lesquels chaque la combinaison d'un premier et d'un second réservoir (1105) est dimensionnée de manière à s'étendre sur une zone couvrant sensiblement la totalité d'au moins un canal de la pluralité de canaux (1122).

8. Dispositif selon la revendication 6 dans lequel :
(a) chaque canal (1122) a une première extrémité et une seconde extrémité et dans lequel un premier via (1114) assure la communication fluidique entre la première extrémité de chaque canal et un premier réservoir (1105) et un second via (1114) fournit un fluide communication entre la seconde extrémité de chaque canal et un second réservoir (1105) ; ou
(b) pour chacun de la pluralité de canaux (1122), un premier via (1114) assure la communication fluidique entre un premier réservoir (1105) et une première extrémité du canal, et un second via (1114) assure une communication fluide entre un second réservoir (1105) et une seconde extrémité du canal.

9. Dispositif selon la revendication 1, dans lequel les première et seconde électrodes (1112) sont fabriquées en tant que structures indépendantes du chariot et sont insérées dans des ouvertures du premier segment caddy (1100).

10. Dispositif selon la revendication 1, dans lequel la première électrode (1112) est disposée sur une première patte déformable et la seconde électrode (1112) est disposée sur une seconde patte déformable.

11. Dispositif selon la revendication 10, dans lequel les pattes déformables sont formées dans le premier segment de caddie (1100) ou les pattes déformables sont attachées à une surface du premier segment de caddie.

12. Dispositif selon la revendication 10, comprenant en outre une étiquette fixée à une surface du premier segment de caddie.

13. Dispositif selon la revendication 12, dans lequel les pattes déformables sont formées dans l'étiquette ou les pattes déformables sont attachées à l'étiquette.

14. Dispositif selon la revendication 10, dans lequel les pattes déformables sont déformées pendant la fabrication de telle sorte que, lorsque le premier segment de caddie (1100) est fixé à la première surface du deuxième segment de caddie (1110),
une partie de la première électrode (1112) s'étend vers le bas dans le premier réservoir (1105) et une partie de la deuxième électrode (1112) s'étend vers le bas dans le deuxième réservoir (1105).

15. Dispositif fluidique selon la revendication 5, le dispositif comprenant en outre :
des premier et second évents disposés dans le chariot, adjacents aux premier et second puits, le premier évent en communication fluidique avec le premier réservoir (1105) et le second évent en communication fluidique avec le second réservoir (1105), les évents équilibrant les différences de pression ce résultat lorsqu'un joint qui est apposé sur les premier et deuxième puits se dilate, créant un vide sous le joint.

16. Système pour isoler un ou plusieurs composants d'échantillon d'un matériau d'échantillon après la séparation du matériau d'échantillon en une pluralité de composants d'échantillon, le système comprenant :
un dispositif fluidique comportant des électrodes incorporées, comprenant :
une carte (1130) ; et
un caddie, le caddie comprenant :
un premier segment de caddie (1100) ; et
un second segment de chariot (1110) comprenant une première surface et une seconde surface ;
dans lequel la carte (1130) est fixée à la seconde surface du second segment de caddie de telle sorte que la carte fournisse une surface fermée pour le dispositif ; et
un détecteur en communication sensorielle avec le dispositif ;
un système de direction de fluide ; et
un processeur couplé de manière opérationnelle au détecteur et au système de direction de fluide ;
**caractérisé en ce que** :
le premier segment de caddy (1100) comprend des première et seconde électrodes (1112) ;
le second segment de caddie (1110) comprend des premier et second réservoirs (1105) disposés sur la première surface du deuxième segment de caddie, un canal (1122) disposé sur la seconde surface du second segment de caddie et des premier et second vias (1114) s'étendant entre les première et seconde surfaces pour établir une communication de fluide entre le réservoir (1105) et le canal (1122) ; et
le premier segment de chariot (1100) peut être fixé à la première surface du second segment de chariot (1110) de telle sorte que, une fois fixée, une première partie de la première électrode (1112) est accessible pour le contact électrique sec et une seconde partie du premier l'électrode s'étend dans le premier réservoir (1105) et de telle sorte qu'une première partie de la deuxième électrode est accessible pour un contact électrique sec et qu'une deuxième partie de la deuxième électrode s'étend dans le deuxième réservoir (1105).
